Europäisches Patentamt

European Patent Office

Office européen des brevets

(19)

(11) Veröffentlichungsnummer: **0 415 036 A2**

(12) **EUROPÄISCHE PATENTANMELDUNG**

(21) Anmeldenummer: 90113066.6

(51) Int. Cl.5: **A61B 5/107**

(22) Anmeldetag: 09.07.90

(30) Priorität: 28.08.89 DE 8910258 U

(43) Veröffentlichungstag der Anmeldung:
06.03.91 Patentblatt 91/10

(84) Benannte Vertragsstaaten:
**AT BE CH DE DK ES FR GB GR IT LI LU NL SE**

(71) Anmelder: **AMOENA**
**Medizin-Orthopädie-Technik GmbH**
**Kapellenweg 36**
**W-8201 Raubling(DE)**

(72) Erfinder: **Syrbe, Reinhard, Dipl.-Ing. (FH)**
**Amselstrasse 31**
**W-8201 Rohrdorf/Thansau(DE)**

(74) Vertreter: **Merten, Fritz**
**Tristanstrasse 5**
**W-8500 Nürnberg 40(DE)**

(54) **Vorrichtung zum Erfassen von auf Druck ansprechenden Belastungen an verschiedenen Bereichen des menschlichen Körpers, wie insbesondere an der Sohle eines Fusses.**

(57) Die Erfindung betrifft eine Vorrichtung (1) zum Erfassen von auf Druck ansprechenden Belastungen an dir. Bereichen eines menschlichen Körpers, insb. an der Sohle eines Fußes, welche von Sensoren gebildet wird, von denen jeder von einem elastischen Flachkörper (2) mit sandwichartiger Anordnung von Leitermitteln (3, 4) gebildet wird, von denen ein Leitermittel (3) Noppen (9) aufweist, die durch die Aussparungen (8) im Flachkörper (2) greifen und bei Belastung der Vorrichtung (1) einen Kontakt mit dem darunterliegenden Leitermittel (4) auslösen. Der jeweilige Schwellenwert des Flachkörpers (2) für das Erfassen der jeweiligen Druckbelastung wird über eine elektrische Verbindung zu einem Signalgeber (7) und von da zu einer optischen oder akustischen Anzeige gegeben.

Fig.5

EP 0 415 036 A2

# VORRICHTUNG ZUM ERFASSEN VON AUF DRUCK ANSPRECHENDEN BELASTUNGEN, AN VERSCHIEDENEN BEREICHEN DES MENSCHLICHEN KÖRPERS, WIE INSB. AN DER SOHLE EINES FUSSES

Die Erfindung bezieht sich auf eine Vorrichtung zum Erfassen von auf Druck ansprechenden Belastungen an verschiedenen Bereichen des menschlichen Körpers, wie insb. an der Sohle eines Fußes, bestehend aus mindestens einem, dem jeweiligen Bereich zugeordneten Sensor und mindestens einem, die Druckbelastung nach außen signalisierenden Geber, wobei Sensor und Geber als elektrische Bauteile ausgeführt, sowie der Sensor als Schalter und der Geber als ein, ein akustisches oder optisches Signal gebender Sender ausgebildet sind, und welcher Sensor an dem die Druckbelastung erfassenden Bereich derart eingelegt ist, daß er eine Punkt-und/oder Flächenbelastung innerhalb des zu erfassenden Bereiches aufnimmt.

Es ist in der Medizintechnik allgemein bekannt, nach entsprechenden Verletzungen diverser Körperteile deren Rehabilitation einzuleiten und im Rahmen der Behandlung derselben neben einer Reihe von meßbaren Ergebnissen auch die Möglichkeiten der Druckbelastung entsprechender Körperteile zu erfassen. Die Erfassung entsprechender Druckbelastungen kann auch zur Vorsorge bei einer Reihe von Erkrankungen herangezogen werden, so etwa in der Ulcus-Prophylaxe an einem diabetischem Fuß u. a. Es ist bekannt, daß bei einer derartigen Erkrankung verschiedene Stellen an der Fußsohle ihr Druckempfinden nach und nach verlieren, wodurch sich bei Fortschreiten dieser Krankheit Knochen freilegen können, die bei einem derartigen Fortschritt der Erkrankung zu Amputationen u. a. führen können.

In der orthopädischen Rehabilitation ist es vielfach erwünscht, eine kontrollierte Teilbelastungstherapie durchzuführen, so daß verschiedene Körperteile nur so lange belastet werden, wie dies, ohne Beeinträchtigung der Rehabilitation, möglich erscheint. Es ist bei dieser Behandlungsart erforderlich, sogenannte Sensibilitätsstauungen rechtzeitig zu erkennen und durch Belastung anderer Körperteile so weit zu entlasten, daß die vorher belasteten Körperteile nicht überbeansprucht werden.

Durch die europäische Patentanmeldung 0 286 054 ist ein Schuh für derartige medizinische Zwecke bekannt geworden, bei dem in dessen Schuhsohle Sensoren, sowohl im Fersen-als auch im Ballenbereich eingelegt sind, über die entsprechende Druckbelastungen aufgenommen und nach außen über eine akustische oder optische Signalgabe angezeigt werden. Die hier verwendeten Sensoren sind großflächig ausgelegt und sie sind so in die Schuhsohle eingearbeitet, daß der auf sie drückende Fuß bei Erreichen des jeweils eingestellten Schwellenwertes die entsprechende Belastung nach außen anzeigen. Das Anzeigen der Belastung kann dabei durch akustische oder optische Signale ausgelöst werden, wodurch der jeweilige Patient schnell erkennt, daß die Belastung des betreffenden Körperteiles den für die Behandlung vorgesehenen Schwellenwert überschreitet. Eine bei dieser Vorrichtung verwendete Steuereinrichtung für das Erfassen des jeweiligen Schwellenwertes erlaubt durch Umlegen von verschiedenen Schaltern verschiedene Druckstufen einzustellen, so daß ein Meßbereich, z. B. von 10 bis 60 kp, eingestellt werden kann. Die bei dieser Vorrichtung verwendeten Sensoren für das Erfassen des Schwellenwertes der jeweiligen Druckbelastung sind in einem Silikonkautschuk eingebettet und als sogenannte Druckschalter ausgebildet, die beim Zusammenführen zweier elektrischer Leiterplatten, nach Durchdrücken des Silikonkautschuks, den elektrischen Kontakt herstellen und damit das entsprechende Signal auslösen. Zwar haben sich derartige Vorrichtungen in der Praxis sehr gut bewährt, indessen ist vielfach erwünscht, den jeweiligen Schwellenwert einfacher einstellen zu können, um entsprechend dem Fortschritt der Behandlung die entsprechenden Belastungsänderungen festzulegen.

Hier setzt nun die Erfindung ein, der die Aufgabe zugrunde liegt, eine Vorrichtung dahingehend weiter zu bilden, daß mit dieser exakte Schwellenwerte eingestellt werden können und entsprechend dem Fortschritt der Behandlung die einzelnen Körperteile mit zunehmenden Druckbelastungen belastet und dennoch kontrolliert bleiben können.

Gemäß der Erfindung wird diese Aufgabe bei einer Vorrichtung der eingangs genannten Art dadurch gelöst, daß der Sensor von einem gitterförmig ausgestatteten, elastischen Flachkörper und diesen sandwichartig begrenzenden Leiterkörpern besteht, von denen mindestens der eine Leiterkörper mit in die gitterförmigen Aussparungen des Flachkörpers eintauchenden Noppen ausgestattet, und der andere Leiterkörper als eine plattenförmige, an dem Flachkörper liegende Leiterplatte ausgebildet ist, und daß die Noppen des einen Leiterkörpers bei entlastetem Flachkörper mit Abstand zur Leiterplatte, bei Belastung des Flachkörpers hingegen auf die Leiterplatte zur Anlage kommen, und daß der Grad der zu erfassenden Druckbelastung am zu messenden Bereich von der Elastizität des Flachkörpers bestimmt ist, wie auch die Belastung selbst durch eine Versorgung der Leiterplatte und des Leiterkörpers mit elektrischem Strom erfaßbar und elektrisch nach außen signalisierbar ist.

Durch diese Maßnahmen wird nicht nur die der Erfindung zugrunde liegende Aufgabe vorteilhaft gelöst, sondern es wird noch eine Reihe weiterer Vorteile erzielt. Durch die Anwendung des elastischen Flachkörpers als Indikator der jeweils zulässigen Druckbelastung läßt sich ein breites Band unterschiedlicher Belastungen für verschiedene Anwendungsfälle, allein durch die Wahl des jeweiligen Materials und damit der Shorehärte des betreffenden Flachkörpers, festlegen. Außerdem hat dieses Material die Eigenschaft, daß es selbst bei unterschiedlichen Temperaturen seine Elastizität beibehält, wodurch Änderungen der betreffenden Schwellenwerte nicht eintreten. Der Druck, der nun direkt auf den Flachkörper bei entsprechender Belastung desselben einwirkt, muß nicht über andere Materialien länger geführt werden, sondern wirkt direkt auf diesen Flachkörper ein, so daß die entsprechende Belastung sofort erfaßt und bei Überschreiten deren Schwellenwertes nach außen signalisiert wird. Die das Signal nach außen übermittelnden Teile sind elektrische Bauteile in Form von flächigen Platten und solchen mit Noppen versehene, die nach Art eines Schalters funktionieren und bei entsprechender Kontaktbringung das Signal auslösen. Die Leiterkörper mit Noppen, wie auch die Leiterplatten sind über Drähte an einer Stromversorgung angeschlossen und sie sind über weitere, in der Zeichnung nicht mehr dargestellte Verbindungen mit dem jeweiligen akustischen oder optischen Signalgeber verbunden was zur Folge hat, daß kaum Behinderungen für den Patienten durch die Versorgungsmittel auftreten.

Gemäß einer vorteilhaften Weiterbildung der Erfindung ist die Vorrichtung in einer Einlegesohle eines Schuhs integriert, und es ist diese Einlegesohle von mindestens zwei, deckungsgleich aufeinanderliegenden Sohlenflächen gebildet, wie auch die Vorrichtung zwischen diesen Sohlenflächen eingelegt ist und flächenförmig den zu erfassenden Bereich der jeweils zu messenden Druckbelastung abdeckt.

Durch diese Ausführung der Vorrichtung lassen sich Sensoren herstellen, die sehr flach ausgeführt werden können und die zwischen Sohleflächen, beispielsweise einer Einlegesohle eingelegt sein können, wodurch sie kaum über die Fläche dieser Einlegesohlen hervorragen.

Eine weitere, vorteilhafte Weiterbildung der Vorrichtung zeichnet sich dadurch aus, daß mindestens ein Sensor im Bereich der Ferse und mindestens ein weiterer Sensor im Bereich des Ballens und/oder der Zehen eines Fußes zwischen den Sohleflächen eingelegt ist.

Auf diese Weise lassen sich, beispielsweise an einer Sohle an verschiedenen Bereichen Druckbelastungen messen, so daß sowohl der sogenannte Abrollvorgang des Gehens, als auch das direkte, vollflächige Aufsetzen des Fußes erfaßt werden können.

Um möglichst viele Stellen innerhalb eines Bereichs zu erfassen, können nach einer Weiterbildung der Vorrichtung deren Sensoren flächendeckend ausgeführt sein, und es können die jeweiligen, elastischen Flachkörper annähernd in deren gesamten Ausdehnung die gitterförmige Struktur aufweisen.

Weitere vorteilhafte Weiterbildungen der Erfindung können insb. den verbleibenden Unteransprüchen, für die im Rahmen des Hauptanspruches Schutz begehrt wird, entnommen werden.

In der Zeichnung ist eine der möglichen Ausführungen einer derartigen Vorrichtung als Sensor selbst und am Beispiel dessen Anwendung bei einer Einlegesohle schematisch dargestellt. Es zeigt:

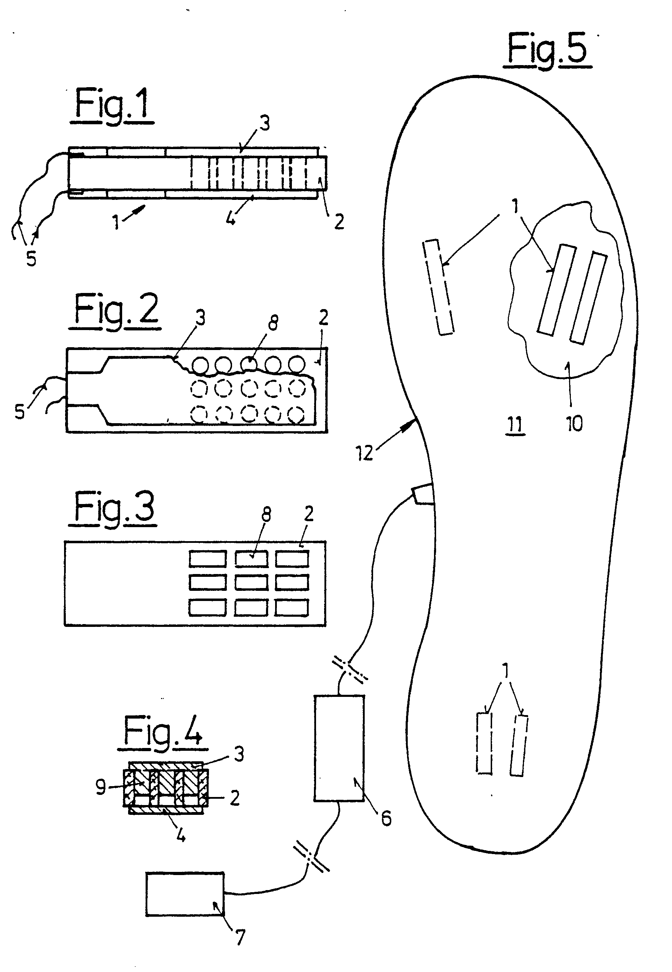

Fig. 1 eine vergrößerte Seitenansicht auf eine Vorrichtung mit dem elastischen Flachkörper und den diesen sandwichartig begrenzenden Leiterplatte und Leiterkörper, von denen letzterer mit durch den Flachkörper dringenden Noppen ausgeführt ist,

Fig. 2 eine Draufsicht auf die Vorrichtung nach Fig. 1, mit kreisrunden Aussparungen für die Noppen des Leiterkörpers,

Fig. 3 eine Draufsicht auf den Flachkörper mit rechteckigen Aussparungen für entsprechende Noppen, jedoch ohne den darüber liegenden Leiterkörper,

Fig. 4 einen Schnitt durch die Vorrichtung in der Ebene IV-IV in Fig. 1,

Fig. 5 eine Draufsicht auf eine Einlegesohle mit den in dieser eingelegten Vorrichtungen bzw. deren Sensoren im Bereich der Ferse und des Ballens eines Fußes, nebst den den Leiterkörper und die Leiterplatte mit elektrischer Energie versorgenden Leitungen und den die Druckbelastung nach außen signalisierenden Geber.

Die Vorrichtung 1 gemäß der Erfindung wird im wesentlichen von einem elastischen Flachkörper 2 und zwei diesen nach oben und unten abdeckenden Leitermitteln 3, 4 gebildet, welche über entsprechende Leitungen 5 sowohl an einer elektrischen Energieversorgung 6 als auch einem Signalgeber 7 angeschlossen sind. Der Flachkörper 2, der aus dem elastischen Werkstoff, z. B. einem Gummi oder Kunststoff (Polyurethan), bestehen kann, ist über annähernd seine gesamte Ausdehnung mit einer Reihe von Aussparungen 8 versehen, und es ist ein Leitermittel als eine flache Leiterplatte 4 und das andere, vorzugsweise darüberliegende Leitermittel 3 als ein, mit Noppen 9 versehener Leiterkörper 3, ausgebildet. Die Noppen 9 dieses Leiterkörpers 3 tauchen in die gitterförmigen Aussparungen 8 des Flachkörpers 2 so weit ein, daß sie vor Belastung dieses Flachkörpers die darunterliegende Leiterplatte 4 noch nicht berühren. Erst bei Belastung dieses Flachkörpers 2 senken sich die einzelnen Noppen 9 in die gitterförmigen Aussparungen 8 des Flachkörpers ein und sie senken sich dabei so weit, daß sie auf die darunterliegende Leiterplatte 4 zur Anlage kommen. Durch diese Anlage der Noppen 9 auf die darunterliegende Leiterplatte 4 wird ein Kontakt geschaffen und damit ein Stromfluß ausgelöst, der zur Folge hat, daß dieser über den Signalgeber 7 die entsprechende Belastung und damit den eingestellten Schwellenwert nach außen anzeigt. Während der Flachkörper 2 aus dem elastischen Werkstoff besteht, können die Leitermittel 3, 4 aus einer Metallfolie oder auch aus einem gummiähnlichen Werkstoff bestehen, der oberflächenseitig mit einer stromleitenden Schicht bedampft bzw. versehen ist. Um diese Leitermittel 3, 4 dabei so flach wie möglich auszuführen und dennoch deren Elastizität über einen längeren Wirkungsbereich aufrecht zu erhalten, empfiehlt es sich, die Leitermittel aus einem Kunststoff herzustellen und diesen mit der stromleitenden Schicht zu versehen, die den entsprechenden Stromfluß leitet.

Die Vorrichtung 1 selbst, bestehend aus dem Flachkörper 2 und den an diesem angebrachten Leitermitteln 3, 4 ist in der Regel sehr flach ausgeführt, wobei deren Stärke von dem jeweiligen Schwellenwert einerseits und den zu erfassenden Druckbelastungen andererseits bestimmt wird. Bei Anwendung dieser Vorrichtung 1 für die Erfassung von Druckbelastungen eines Fußes empfiehlt es sich, die Vorrichtung zwischen die Sohlenhälften 10, 11 einer Einlegesohle 12 einzubringen und diese Vorrichtung 1 so flach auszubilden, daß nach deren Einlegung in den Sohlenhälften diese kaum über die Fläche der Einlegesohle 12 ragt. Aufgrund dieser Ausgestaltung der Vorrichtung 1 ist es möglich, diese an verschiedenen Bereichen einer Einlegesohle 12 vorzusehen, so daß beispielsweise der Bereich der Ferse ebenso erfaßt werden kann, wie z. B. der Bereich des Ballens eines Fußes, wodurch sowohl Rollbewegungen des Fußes als auch abrupte, senkrechte Belastungen desselben erfaßt werden können. Der verwendete Signalgeber für das Anzeigen der entsprechenden Druckbelastung nach außen kann ein akustischer oder optischer Signalgeber sein, wobei es selbstverständlich nicht ausgeschlossen ist, auch eine Funkübertragung der gemessenen Schwellenwerte vorzunehmen.

Die einzelnen Vorrichtungen 1 können auf einen jeweils bestimmten Schwellenwert eingestellt sein, welcher durch die Härte bzw. Elastizität des Flachkörpers 2 bestimmt wird, und es können in einem solchen Fall Einlegesohlen 12 mit unterschiedlichen Vorrichtungen 1 im Bezug auf deren Schwellenwerte verwendet werden, so daß entsprechend der Behandlung eines Patienten mit einem und dem gleichen Schuh verschiedene Einlegesohlen wahlweise, getragen werden können.

Auf diese Weise lassen sich die einzelnen Belastungen, z. B. die eines Fußes, während einer Therapie kontinuierlich erfassen und auswerten, so daß mit zunehmender Gesundung des jeweiligen Körperteiles die Schwellenwerte der Belastung angehoben werden können.

Die Erfindung wurde hier am Beispiel einer Einlegesohle 12 für das Erfassen der Druckbelastungen, insb. für die Durchführung einer Teilbelastungstherapie nach Eingriffen an der unteren Extremität, z. B. nach Totalhüftendoprothese, gelenknahen Knochenbrüchen usw., dargestellt und beschrieben. Dies schließt natürlich nicht aus, solche Vorrichtungen 1 auch in der Ulcusprophylaxe am diabetischen Fuß oder für das Erfassen anderer Belastungen, an anderer Körperteile zu verwenden, so beispielsweise der Beuge eines Knies oder Armes oder dem Erfassen einer Druckbelastung im Bereich des Gesäßes usw.

Das Früherfassen der jeweiligen Belastung einerseits und das Kontrollieren derselben während des Behandlungsvorganges andererseits sind Mitgaranten für eine optimale Behandlung und dürften in der Behandlung diverser Erkrankungen unentbehrlich sein.

## BEZUGSZEICHEN-LISTE

1 Vorrichtung
2 Flachkörper
3 Leiterkörper
4 Leiterkörper
5 Leitung
6 Energieversorgung
7 Signalgeber
8 Aussparung
9 Noppen
10 Sohlenhälften
11 Sohlenhälften
12 Einlegesohle

## Ansprüche

1. Vorrichtung zum Erfassen von auf Druck ansprechenden Belastungen an verschiedenen Bereichen des menschlichen Körpers, wie insb. an der Sohle eines Fußes, bestehend aus mindestens einem, dem jeweiligen Bereich zugeordneten Sensor und mindestens einem, die Druckbelastung nach außen signalisierenden Geber, wobei Sensor und Geber als elektrische Bauteile ausgeführt, sowie der Sensor als Schalter und der Geber als ein, ein akustisches oder optisches Signal gebender Sender ausgebildet sind, und welcher Sensor an dem die Druckbelastung erfassenden Bereich derart eingelegt ist, daß er eine Punkt- und/oder Flächenbelastung innerhalb des zu erfassenden Bereiches aufnimmt, dadurch gekennzeichnet , daß der Sensor von einem gitterförmig ausgestatteten, elastischen Flachkörper (2) und diesen sandwichartig begrenzenden Leiterkörpern (3, 4) besteht, von denen mindestens der eine Leiterkörper (3) mit in die gitterförmigen Aussparungen (7) des Flachkörpers (2) eintauchenden Noppen (9) ausgestattet, und der andere Leiterkörper (4) als eine plattenförmige, an dem Flachkörper (2) liegende Leiterplatte ausgebildet ist, und daß die Noppen (9) des einen Leiterkörpers (3), bei entlastetem Flachkörper (2) mit Abstand zur Leiterplatte (4), bei Belastung des Flachkörpers (2) hingegen auf die Leiterplatte (4) zur Anlage kommen, und daß der Grad der zu erfassenden Druckbelastung am zu messenden Bereich von der Elastizität des Flachkörpers (2) bestimmt ist, wie auch die Belastung selbst durch eine Versorgung (6) der Leiterplatte (3) und des Leiterkörpers (4) mit elektrischem Strom erfaßbar und elektrisch nach außen signalisierbar ist.

2. Vorrichtung nach Anspruch 1, wobei diese für die Erfassung von Belastungen im Bereich, wie der Sohle eines Fußes, konzipiert ist, dadurch gekennzeichnet , daß diese Vorrichtung (1) in einer Einlegesohle (12) eines Schuhs integriert und diese Einlegesohle von mindestens zwei, deckungsgleich aufeinander liegenden Sohlenflächen (10, 11) gebildet ist, und daß die Vorrichtung (1) zwischen diesen Sohleflächen (10, 11) eingelegt ist und flächenförmig den zu erfassenden Bereich der jeweils zu messenden Druckbelastung an der Sohle abdeckt.

3. Vorrichtung nach Anspruch 2, dadurch gekennzeichnet , daß mindestens ein Sensor (Vorrichtung 1) im Bereich der Ferse und mindestens ein weiterer Sensor im Bereich des Ballens und/oder der Zehen des Fußes zwischen den Sohleflächen (10, 11) eingelegt ist.

4. Vorrichtung nach Anspruch 1, dadurch gekennzeichnet , daß der Leiterkörper (3) mit dessen Noppen (9) und/oder die Leiterplatte (4) aus einem elastischen Werkstoff besteht, und daß dieser Werkstoff zum Zwecke seiner Leitfähigkeit mit einer Strom leitenden Beschichtung (Schicht) versehen ist.

5. Vorrichtung nach Anspruch 2, dadurch gekennzeichnet , daß diese sehr flach ausgeführt ist und möglichst die Stärke der sie aufnehmenden Einlegesohle (12) kaum überschreitet.

6. Vorrichtung nach Anspruch 1, dadurch gekennzeichnet , daß deren Sensoren (Vorrichtung 1) rechteckig ausgeführt sind, und daß die jeweiligen, elastischen Flachkörper (2), annähernd in deren gesamten Ausdehnung, die gitterförmige Aussparung (8) aufweisen.

7. Vorrichtung nach Anspruch 1, dadurch gekennzeichnet , daß deren Sensoren (Vorrichtung 1) rund oder oval ausgeführt sind, und daß die jeweiligen, elastischen Flachkörper (2), annähernd in deren gesamten Ausdehnung, die gitterförmige Aussparung (8) aufweisen.

8. Vorrichtung nach Anspruch 1, dadurch gekennzeichnet , daß jeder Flachkörper (2) auf einen, durch dessen Elastizität festgelegten Schwellenwert der Druckbelastung festgelegt ist, und daß für das Messen der jeweiligen Druckbelastung in unterschiedlichen Bereichen gleiche oder ungleiche Schwellenwerte aufweisende Flachkörper den einzelnen Bereichen zugeordnet sind.

9. Vorrichtung nach Anspruch 7, dadurch gekennzeichnet , daß bei Anwendung derselben in einer Einlegesohle (12) für einen Schuh, die Flachkörper (2) im Bereich der Ferse einen höheren Schwellenwert als die Flachkörper (2) im Bereich des Ballens des Fußes aufweisen.

10. Vorrichtung nach Anspruch 8, dadurch gekennzeichnet , daß jeder Sensor mindestens in zwei Hälften, d. h. eine linke und rechte Hälfte, geteilt ist, und daß die linke Hälfte auf den maximalen Schwellenwert eingestellt ist, hingegen die rechte Hälfte auf einen solchen Wert eingestellt ist, der dem Maximalwert und/oder der Anzahl der eingelegten Sensoren entspricht.

11. Vorrichtung nach Anspruch 9, dadurch gekennzeichnet , daß die linken Hälften der Sensoren (Vorrichtung 9) für das Erfassen der Druckbelastung aus dem Abrollvorgang in einer Oder-Schaltung, und daß die rechten Hälften der Sensoren für das Erfassen der Druckbelastung aus dem vollflächigen Auftreten in einer Und-Schaltung verknüpft sind.

Fig.1

Fig.2

Fig.3

Fig.4

Fig.5